**Europäisches Patentamt**

**European Patent Office**

**Office européen des brevets**

(11) Veröffentlichungsnummer: **0 516 610 B1**

(12) **EUROPÄISCHE PATENTSCHRIFT**

(45) Veröffentlichungstag der Patentschrift: **21.06.95**

(51) Int. Cl.⁶: **G01N 21/64**, G01N 21/63, G01N 21/62

(21) Anmeldenummer: **92890100.8**

(22) Anmeldetag: **27.04.92**

(54) **Vorrichtung zur Bestimmung des Materieflusses.**

(30) Priorität: **10.05.91 AT 976/91**

(43) Veröffentlichungstag der Anmeldung:
**02.12.92 Patentblatt 92/49**

(45) Bekanntmachung des Hinweises auf die
Patenterteilung:
**21.06.95 Patentblatt 95/25**

(84) Benannte Vertragsstaaten:
**AT DE FR GB**

(56) Entgegenhaltungen:
EP-A- 0 198 815        AT-B- 390 300
DD-A- 106 086        DD-A- 142 973
US-A- 4 994 396        US-A- 5 006 314

(73) Patentinhaber: **AVL Medical Instruments AG**
**Stettemerstrasse 28**
**CH-8207 Schaffhausen (CH)**

(72) Erfinder: **Lübbers, Dietrich W., Prof.Dr.Med.**
**Wenkerstrasse 30**
**W-4600 Dortmund (DE)**
Erfinder: **Karpf, Hellfried, Dr.**
**Schwarzbauerweg 25**
**A-8043 Graz (AT)**

(74) Vertreter: **Krause, Walter, Dr. Dipl.-Ing. et al**
**Postfach 200**
**A-1014 Wien (AT)**

**Beschreibung**

Die Erfindung betrifft eine Vorrichtung zur Bestimmung des Materieflusses durch eine Grenzfläche.

Unter Materiefluß sei in diesem Zusammenhang ein Fluß von Partikeln, beispielsweise von Ionen, Molekülen oder Gasen zu verstehen, der durch eine Grenzfläche zwischen unterschiedlichen Medien- bzw. auch unterschiedlichen Phasenzuständen eines Mediums durchtritt und dessen Größe pro Flächen- und Zeiteinheit, beispielsweise in Teilchen pro Sekunde und Quadratmeter oder $mol \ s^{-1} \ m^{-2}$ quantifiziert werden soll.

Wenn es möglich ist, auf beiden Seiten einer Grenzfläche die Partikelkonzentration, den Gaspartialdruck etc. zu messen, so kann aus der Differenz der Meßwerte auf den entsprechenden Materiefluß geschlossen werden. Schwierigkeiten treten dann auf, wenn die nötigen Daten auf einer Seite der Grenzfläche nicht bzw. nicht mit der gewünschten Genauigkeit erfaßt werden können.

Aufgabe der Erfindung ist es, eine Vorrichtung vorzuschlagen, mit welcher der Materiefluß auch dann gemessen werden kann, wenn eine Seite der Grenzfläche nicht oder nur schwer für eine Messung zugängig ist, wenn insbesondere die Grenzfläche eine Organoberfläche oder die Hautoberfläche ist.

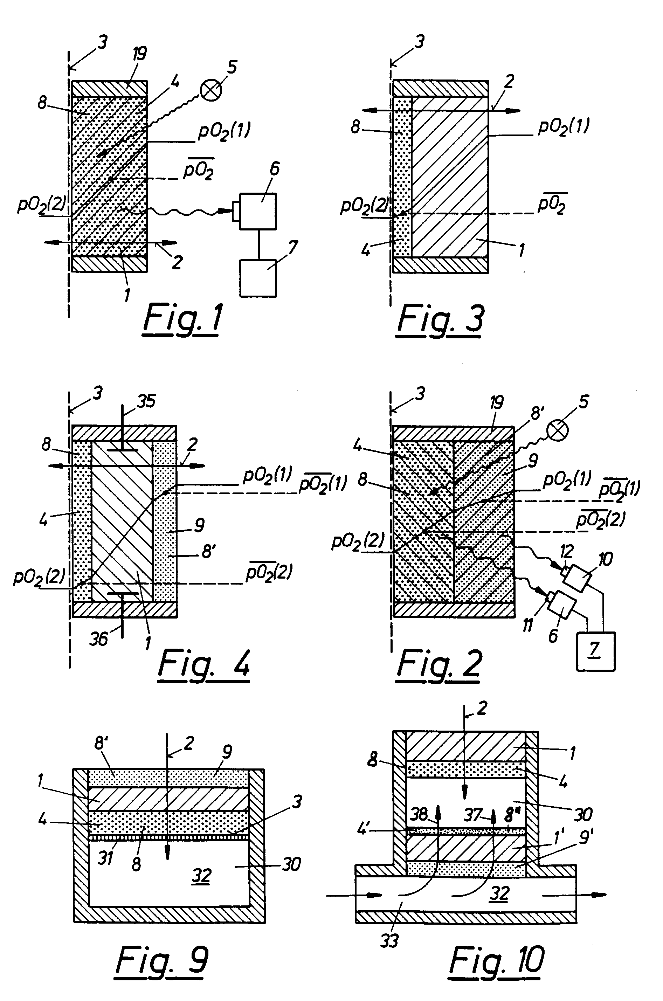

Diese Aufgabe wird erfindungsgemäß dadurch gelöst, daß zumindest eine der Grenzfläche zuordenbare Sensorschicht vorgesehen ist, welche dem Materiefluß einen bekannten oder vorbestimmbaren, endlichen Widerstand entgegensetzt, daß in der Sensorschicht ein optischer Indikator zur Bestimmung der ersten Meßwertes einer von der durchschnittlichen Materiekonzentration in der Sensorschicht abhängigen Größe vorgesehen ist, daß ein zweiter Meßwert dieser Größe an einer Seite der Sensorschicht bekannt oder durch einen weiteren, in einer zweiten Sensorschicht vorliegenden optischen Indikator bestimmbar ist, sowie daß eine Auswerteeinrichtung zur Bestimmung des Materieflusses aus der Differenz der beiden Meßwerte vorgesehen ist. Durch die erfindungsgemäße Vorrichtung erfolgen alle Konzentrationsmessungen auf einer Seite der Grenzschicht, wodurch die eingangs genannten Schwierigkeiten bewältigt werden können. Das Meßprinzip wird in der Figurenbeschreibung anhand von vier grundlegenden Vorrichtungsvarianten Fig. 1 bis 4 näher erläuert. In den der Grenzfläche zuordenbaren Schichten dürfen die zu messenden Partikel bzw. Moleküle weder erzeugt noch vernichtet (z.B. durch chemische Reaktionen) oder angelagert werden, um das Meßergebnis nicht zu verfälschen.

Falls nicht die Sensorschicht gleichzeitig auch als Widerstandsschicht verwendet wird, kann in einer Weiterbildung der Erfindung vorgesehen sein, daß zumindest eine an der Sensorschicht anliegende bzw. zwischen den beiden Sensorschichten angeordnete Widerstandsschicht vorgesehen ist, welche dem Materiefluß einen bekannten oder vorbestimmbaren, endlichen Widerstand entgegensetzt. Es kann dann die Sensorschicht sehr dünn und für den Materiefluß praktisch widerstandslos ausgeführt sein, z.B. kann deren Indikator (z.B. ein lumineszenzoptischer Indikator) direkt an der Widerstandsschicht immobilisiert sein.

Als Schichtmaterial für die Widerstandsschicht eignet sich z. B. Hydrogel, Latex, Controlled Pore Glass (CPG), Zellophan oder eine Nukleopormembran. Als Material für die Sensorschicht eignen sich beispielsweise PVC, Polyurethan, Silikon, Hydrogel, Latex, Cellulose.

Als Indikatoren seien auch nur beispielhaft für die Bestimmung des $O_2$-Flusses Pyrenbuttersäure, Decacyclen sowie Ruthenium- und Osmiumkomplexe (Bipyridil, Phenanthrolin) genannt. Für den $H^+$-Ionenfluß bzw. den $CO_2$-Fluß kann Hydroxipyrentrisulfonsäure zur Anwendung kommen.

Beispielsweise kann erfindungsgemäß vorgesehen sein, daß zur Bestimmung des Gasflusses alle Sensor- bzw. Widerstandsschichten zumindest für das zu messende Gas durchlässig sind, wobei zumindest einer der optischen Indikatoren zur Bestimmung einer vom Gaspartialdruck oder der Gaskonzentration abhängigen Größe geeignet ist.

Eine besonders vorteilhafte Anwendung dieser Art ist dadurch gegeben, daß zur Bestimmung des Flusses zumindest eines der Gase $O_2$, $CO_2$, $H_2$, $NH_3$, Wasserdampf oder Narkosegase durch eine Organoberfläche, z.B. durch die Hautoberfläche als Grenzfläche, eine an beiden Seiten offene, auf die Organoberfläche mit einer Seite aufsetzbare Halterung vorgesehen ist, in welcher die gasdurchlässigen Sensor- bzw. Widerstandschichten angeordnet sind, wobei die Sensorschicht zur Bestimmung des Gaspartialdruckes mit einer Seite an der Organoberfläche anliegt und mit der anderen Seite ggf. unter Zwischenlage einer Widerstandsschicht mit einem Gasreservoir bekannter Gaszusammensetzung verbindbar ist. So ist beispielsweise für die Messung des $O_2$- oder $CO_2$-Flusses durch die Haut nur eine Sensorschicht notwendig, da der $O_2$- bzw. $CO_2$-Gehalt der Luft für viele Anwendungsfälle als genügend konstant betrachtet werden kann, um in die Auswertung direkt als Konstante eingehen zu können.

Weiters ist es im Zusammenhang mit der Durchflußmessung an Organen, z.B. der Haut von Vorteil, wenn die Halterung im Bereich von der der Organoberfläche abgewandten Seite durch einen Verschluß gasdicht verschließbar ist. Nach dem Verschließen der Halterung wird z.B. der in der Sensorschicht und eventuell in der Widerstandsschicht gespeicherte Sauerstoff durch die Haut aufgenommen, wodurch der

$O_2$-Partialdruck an der Hautoberfläche abnimmt. Aus der Geschwindigkeit der Druckänderung kann auf die Durchblutung des vermessenen Hautbereiches geschlossen werden.

Es ist in diesem Zusammenhang von Vorteil, wenn die zumindest eine Sensorschicht sowie eine allfällige Widerstandsschicht in einer thermostatisierbaren Halterung angeordnet sind, bzw. wenn zumindest eine der Sensor- und/oder Widerstandsschichten aus einem elektrisch leitenden Polymer besteht, welches über elektrische Anschlüsse verfügt, wodurch eine Widerstandsheizung realisierbar ist. Über die Messung der Stromaufnahme für die Thermostatisierung kann ebenfalls auf die Durchblutung des vermessenen Hautbereiches geschlossen werden.

Weiters ist es in diesem Zusammenhang erfindungsgemäß möglich, daß als Gasreservoir eine auf die Halterung aufsetzbare, ggf. thermostatisierbare Begasungseinrichtung vorgesehen ist. Damit kann z.B. an der der Hautoberfläche abgewandten Seite der Polymerschicht ein vom Gasgehalt der Luft unterschiedlicher $pCO_2$ bzw. $pO_2$ angelegt werden. Beispielsweise kann auch reiner Sauerstoff, ein Inertgas oder ein Gas das die Austauschvorgänge an der Hautoberfläche beeinflußt, angeboten werden, wodurch der jeweilige Gasfluß als Funktion des außen anliegenden Gaspartialdruckes bestimmt werden kann und so weitere Parameter über die Gaskinetik der Haut erhältlich sind.

Vorteilhafterweise läßt sich die Thermostatisierung bzw. Widerstandsheizung dadurch regeln, daß zumindest eine der Sensorschichten zusätzlich einen optischen Indikator zur Messung der Temperatur aufweist. Alle hier beschriebenen Temperaturmeß- bzw. Heizeinrichtungen sind natürlich nicht auf die Gasflußmessung an Organoberflächen oder der Haut beschränkt, sondern sind auch auf die noch folgenden Meßsituationen anwendbar.

Die der Erfindung zugrundeliegende Idee ist auf unterschiedlichsten Gebieten anwendbar. So können z.B. zur Bestimmung des Ionenflusses alle Sensor- bzw. Widerstandsschichten ionenpermeabel sein, wobei der zumindest in der einen Sensorschicht vorliegende optische Indikator zur Bestimmung einer von der Ionenkonzentration abhängigen Größe geeignet ist. Beispielsweise ist es mit einer derartigen Vorrichtung möglich, den Ionenaustausch zwischen einer Nährlösung und einem darin gelagerten Spenderorgan zu messen und so Rückschlüsse auf den Zustand des Organs zu erhalten, ohne Proben entnehmen zu müssen. Vorteilhafterweise kann dabei die Sensor- und ggf. die Widerstandsschicht einen Ionencarrier für die Messung eines Ions aus der Gruppe $Na^+$, $K^+$, $Li^+$, $Mg^{2+}$, $Ca^{2+}$, $Cl^-$, $NH_4^+$ aufweisen.

Die Ionenkonzentration kann dabei z.B. nach einem aus der US-PS 4 892 640 bekannten Verfahren bestimmt werden. Hier befindet sich in einer PVC-Membran ein Ionencarrier, sowie ein geladener Fluorophor, der bei Einbringen einer geladenen Spezies (Elektrolyt) zur Aufrechterhaltung der Elektroneutralität der Membran aus dieser gedrückt wird. In der Folge kommt es an der Grenzschichte des Sensors zu sehr hohen Fluorophor-Konzentrationen und zur Konzentrationslöschung der Fluoreszenz.

Weiters kann in einer Ausgestaltung der Erfindung zur Bestimmung des Flusses eines an einer biochemischen Reaktion beteiligten (verbrauchten bzw. erzeugten) Gases ein Reaktionsraum vorgesehen sein, welcher ein biochemisches Substrat, vorzugsweise ein Enzym, enthält und mit der Sensorschicht sowie zumindest indirekt mit einer Probe in Kontakt steht, weiche ein zu bestimmendes Agens, vorzugsweise ein entsprechendes Enzymsubstrat enthält, wobei in Abhängigkeit des von der Auswerteeinrichtung festgestellten Wertes für den Gasfluß die davon abhängige Konzentration des Agens in der Probe bestimmbar ist.

In einer Ausführungsvariante mit einem als Durchflußzelle ausgeführten Probenraum ist vorgesehen, daß zwischen dem Reaktionsraum und einem Probenraum zumindest eine weitere Sensorschicht und ggf. eine weitere Widerstandsschicht angeordnet sind, wobei die Sensorschicht und die Widerstandsschicht sowohl für das an der biochemischen Reaktion beteiligte Gas als auch für das entsprechende Agens in der Probe durchlässig sind.

Der Reaktionsraum kann z.B. eine Zellkultur enthalten, deren Reaktion auf einen in der Probe enthaltenen toxischen Stoff über den Gasstoffwechsel der Zellkultur gemessen wird.

Der Reaktionsraum kann in einer weiteren Variante der Erfindung z.B. das Enzym Glucoseoxydase (GOD) zur Messung der Glucosekonzentration bzw. das Enzym Lactatdehydrogenase oder Lactatoxigenase zur Messung der Lactatkonzentration in einer Probe aufweisen, wobei die bzw. jede Sensorschicht einen Indikator zur Bestimmung des $pO_2$-Wertes aufweist. Weitere Beispiele für dieses Meßprinzip sind untenstehend angeführt:

EP 0 516 610 B1

| Enzym | Enzymsubstrat |
|---|---|
| Xantinoxydase | Hypoxantin |
| Alkoholoxydase | Ethanol |
| Lactatmonooxigenase | Lactat |
| Glyceroldehydrogenase | Glycerol |

Vorteilhaft kann das biochemische Substrat bzw. das Enzym in einer Schicht vorliegen, welche an der dem Reaktionsraum zugewandten Seite der Sensorschicht immobilisiert ist.

Um die Meßempfindlichkeit der Vorrichtung zu erhöhen, bzw. die Messung störende Substanzen am Zutritt zur immobilisierten Schicht zu hindern, kann die Schicht mit dem biochemischen Substrat auf der dem Reaktionsraum zugewandten Seite eine permeabilitätsbestimmende Membran aufweisen.

Eine weitere Anwendungsmöglichkeit ist dadurch gegeben, daß zur Bestimmung des Flusses eines Enzymsubstrates alle Sensor-bzw. Widerstandsschichten für die an einer enzymatischen Reaktion beteiligten Enzymsubstrate durchlässig sind, wobei die zumindest eine Sensorschicht zur Messung der Konzentration eines der Enzymsubstrate ein Enzym aus der Gruppe der Oxydasen und Oxigenasen mit dem dazugehörigen Flavincoenzym (FMN, FAD) aufweist, dessen Eigenfluoreszenz (intrinsische Fluoreszenz) von der Konzentration des Enzymsubstrates abhängig ist. Mit dieser Ausführungsvariante der erfindungsgemäßen Vorrichtung kann beispielsweise der Glucosefluß zwischen einer Nährlösung und der von dieser versorgten Kultur gemessen werden, wenn der Grenzfläche zwischen Nährlösung und Kultur eine oben beschriebene Vorrichtung zugeordnet wird.

Obwohl alle bekannten Sensorschichten in der erfindungsgemäßen Vorrichtung verwendet werden können, solange sie für die zu mossenden Partikel bzw. Moleküle durchlässig sind, wobei auch alle optischen Meßprinzipien wie Absorptionsmessen, Reflexionsmessung etc. - zulässig sind, ist es von Vorteil, wenn die Sensorschicht (Optode) einen lumineszenzoptischen Indikator enthält.

Weiters ist es besonders für jene Anwendungsfälle, wo die eigentliche erfindungsgemäße Vorrichtung am Ende eines Lichtleiters angeordnet ist, notwendig, daß die Sensorschicht und die Widerstandsschicht für die Anregungsstrahlung und/oder die vom Indikator emittierte Strahlung durchlässig sind.

Schließlich ist es insbesondere für jene Fälle, wo die Sensorschicht für Enzymsubstrate durchlässig sein muß, von Vorteil, wenn der optische Indikator in Nanokapseln angeordnet ist, welche in der Sensorschicht gleichmäßig verteilt sind.

Häufig besteht bei der Messung des Materieflusses die Aufgabe, in einem größeren Bereich die Stelle (oder Stellen) zu finden, an denen der Fluß, z.B. von Sauerstoff, gestört ist. Hierzu sollte die räumliche, d. h. die topographische Verteilung der Flußwerte gemessen werden. Dies ist z.B. an der Haut von großer Wichtigkeit, weil mit einem entsprechenden $O_2$-Flußsensor jene Stellen aufspürbar wären, an denen sich eine Mikrozirkulationsstörung ausbildet.

Um dies zu erreichen, ist erfindungsgemäße eine mit der Auswerteeinrichtung verbundene Meßvorrichtung vorgesehen, welche die zumindest eine Sensorschicht zur Erfassung der topographischen Verteilung des Materieflusses flächenförmig abtastet. Die topographische Auflösung der Vorrichtung hängt von den Diffusionseigenschaften ihrer Sensorschichten ab, da bei topographisch unterschiedlichen Konzentrationen (Drucken) eine Querdiffusion innerhalb der Sensorschicht auftreten kann, wodurch die topographische Auflösung verschlechtert wird.

Dies läßt sich jedoch erfindungsgemäß dadurch verhindern, daß die Diffusionseigenschaften der Sensorschicht an jene des zu messenden Objektes angepaßt werden. Um eine möglichst hohe topographische Auflösung zu erreichen, sollten die Diffusionskoeffizienten der Sensorschichten deutlich kleiner sein als jene des zu messenden Objektes.

Eine weitere Verbesserung der topographischen Auflösung läßt sich schließlich dadurch erreiche, daß die Sensorschicht in ein die Querdiffusion in der Sensorschicht verhinderndes Netzwerk z.B. aus Metall, Glas oder spezielle Kunststoffe mit einem hohen Diffusionskoeffizienten eingebettet ist. Die Maschenweite des Netzes bestimmen dann die topographische Auflösung.

In einer besonderen Ausführung kann das Netzwerk z.B. aus Metalldraht oder aus Lichtleitern bestehen, und z.B. zur Thermostatisierung des Flußsensors verwendet werden. Die Lichtleiter lassen sich auch als Teil der optischen Meßanordnung verwenden, beispielsweise um das Anregungslicht in die Sensorschicht einzuleiten.

Häufig besteht beispielsweise in der Medizin die Aufgabe, mehrere Parameter gleichzeitig zu erfassen, um so zu einer besser gesicherten Diagnose zu kommen. Es ist z.B. von Vorteil, wenn nicht nur der lokale Sauerstofffluß und Sauerstoffdruck, sondern auch durch Zusatzgeräte möglichst an der gleichen Stelle die $O_2$-Sättigung des Hämoglobins (durch Messung mit einem Oximeter) und das Verhalten der Erytrozyten-

4

flußgeschwindigkeit (z.B. durch Messen nach dem Laser-Doppler-Verfahren) gemessen werden kann. Um dies zu erreichen, lassen sich die Sensorschichten bzw. Widerstandsschichten so ausbilden, daß sie für die Meßwellenlängen der optischen Zusatzgeräte transparent sind. Ebenso können die Indikatoren so ausgewählt werden, daß sie die optische Analyse in den Zusatzgeräten nicht stören. Da die verschiedenen Meßgrößen durch unterschiedliche optische Signale charakterisierbar sind (z.B. durch unterschiedliche spektrale Emission oder Absorption) können sie auch bei gleichzeitiger Erfassung durch eine Mehrkomponentenanalyse getrennt analysiert werden.

Die vorliegende Erfindung wird im folgenden anhand von schematischen Zeichnungen näher erläutert. Es zeigen:

| Fig. 1 | eine erfindungsgemäße Vorrichtung zur Bestimmung des Materialflusses durch eine Grenzfläche, |
| Fig. 2 bis 4 | Ausführungsvarianten nach Fig. 1, |
| Fig. 5 | eine Ausführungsvariante nach Fig. 4, |
| Fig. 6 | eine Variante zur Bestimmung des Gasaustausches durch die Hautoberfläche, |
| Fig. 7 | eine Zusatzeinrichtung zur Ausführung nach Fig. 6, |
| Fig. 8 | eine Ausführungsvariante zur Messung des Ionenflusses, |
| Fig. 9 und 10 | eine Ausführungsvariante zur Messung der Konzentration eines Enzymsubstrates, sowie |
| Fig.11 | eine Vorrichtung zur Messung der topographischen Verteilung des Materieflusses. |

Die in Fig. 1 dargestellte Vorrichtung weist eine Sensorschicht 4 auf , welche dem durch den Pfeil 2 angedeuteten Materiefluß durch die Grenzfläche 3 einen bekannten, vorbestimmbaren, jedoch endlichen Widerstand entgegensetzt. Die Sensorschicht 4 weist einen optischen Indikator 8 auf, mit welchem die Konzentration der Materie (z.B. Sauerstoff), deren Fluß bestimmt werden soll, gemessen wird. Der Indikator 8 muß dazu zumindest eine seiner optischen Eigenschaften in Abhängigkeit von der Materiekonzentration ändern. Die Anregung des optischen Indikators 8 erfolgt über eine Lichtquelle 5, wobei zwischen Lichtquelle 5 und Detektor 6 hier nicht weiter dargestellte Filtereinrichtungen vorgesehen sein können. Das von der Sensorschicht 4 bzw. vom Indikator 8 emittierte Licht gelangt zu einem Detektor 6, welcher mit einer Auswerteeinrichtung 7 in Verbindung steht. Als Indikator kann beispielsweise ein lumineszenzoptischer Indikator verwendet werden, welcher in der Sensorschicht in bekannter räumlicher Verteilung chemisch oder physikalisch immobilisiert ist. Seitlich kann die Sensorschicht 4 von einer Halterung 19 begrenzt sein.

Die Sensorschicht 4 bildet bei dieser einfachen Ausführungsvariante gleichzeitig eine Widerstandsschicht 1 für den zu messenden Materiefluß, Anhand beispielsweise des $O_2$-Flusses durch die Grenzfläche 3 kann das Meßprinzip folgendermaßen erklärt werden:

Ausgehend von einem bekannten Sauerstoffpartialdruck $pO_2(1)$, beispielsweise der Umgebungsluft, und einem unbekannten $pO_2(2)$ jenseits der Grenzschicht 3, stellt sich in der Sensorschicht ein von den Schichtparametern und der Schichtdicke abhängiger Partialdruck ein, dessen mittlerer Druck $\overline{pO_2}$ vom Detektor 6 erfaßt wird.

Für den $O_2$-Fluß $J(O_2)$ gilt:

$$J(O_2) = P_1(pO_2(1) - pO_2(2)) = P_2(pO_2(1) - \overline{pO_2}) \qquad (1)$$

mit $P_1 = \alpha.D/d$ und $P_2 = K_1.P_1$

| $\alpha$ | Löslichkeitskoeffizient |
| D | Diffusionskoeffizient |
| d | Dicke der Schicht |
| $K_1$ | Verteilungskoeffizient des Indikators |

Falls die Konzentration der jeweiligen Materie auf einer Seite der Sensorschicht 4 bekannt ist, genügt somit in der einfachsten Ausführung der Erfindung bereits eine Sensorschicht, um aus der Differenz zwischen einem Meßwert $(\overline{pO_2})$ und einer Konstanten $(pO_2(1)$ den Materiefluß $J(O_2)$ zu bestimmen.

Entsprechend Fig. 2 ist für den Fall, daß die Materiekonzentration an beiden Seiten der Grenzfläche 3 variiert, parallel zur ersten Sensorschicht 4 eine zweite Sensorschicht 9 mit einem vom ersten Indikator 8 unterschiedlichen Indikator 8' vorgesehen. Die Sensorschichten können, müssen aber nicht aus unterschiedlichen Materialien bestehen. Beispielsweise der $O_2$-Fluß kann damit folgendermaßen bestimmt werden:

$$J(O_2) = P_3(\overline{pO_2}(1) - \overline{pO_2}(2)) \qquad (2)$$

$P_3$ ist eine Konstante, welche von den Parametern der Sensorschichten 4 und 9 sowie von den Verteilungskoeffizienten der Indikatoren 8, 8' und allfälligen Geometriefaktoren der Meßanordnung abhängt.

In den Fig. 3 und 4 sind den Fig. 1 und 2 entsprechende Ausführungsvarianten dargestellt, bei welchen eine an der Sensorschicht 4 anliegende Widerstandsschicht 1 (Fig. 3) bzw. eine zwischen den beiden Sensorschichten 4 und 9 angeordnete Widerstandsschicht 1 vorgesehen ist. Die Sensorschichten 4 und 9 übernehmen hier nur die Meßfunktionen, können sehr dünn ausgeführt werden und sollen dem Materiefluß praktisch keinen Widerstand entgegensetzen, sodaß gilt:

$$\overline{pO_2}(1) \sim pO_2(1) \text{ und } \overline{pO_2}(2) \sim pO_2(2)$$

Die Widerstandsschicht 1 ist für die Ausbildung der Gradienten der Stoffkonzentrationen verantwortlich, sodaß dessen Parameter $\alpha$, D und die Dicke d bekannt oder bestimmbar sein müssen. Ansonsten gelten auch hier bei der Bestimmung des Flusses die Formeln (1) und (2) mit entsprechend geänderten Konstanten.

Zur Thermostatisierung kann eine der Schichten (in Fig. 4 z.B. die Widerstandsschicht 1) aus einem elektrisch leitenden Polymer bestehen, wobei über elektrische Anschlüsse 35 und 36 eine Widerstandsheizung realisierbar ist.

Bei der Anordnung nach Fig. 1 muß die Sensorschicht 4 gleichzeitig auch für die Anregungsstrahlung und für die vom Indikator 8 emittierte Strahlung durchlässig sein.

Weiters müssen die Strahlwege der Sensorschichten 4 bzw. 9 zu den jeweiligen Detektoren 6 und 10 der Ausführung nach Fig. 2 optisch getrennt geführt werden, oder es müssen unterschiedliche Indikatormaterialien 8 und 8' verwendet werden, deren Strahlung durch Eingangsfilter 11 und 12 an den Detektoren 6 und 10 separiert wird. Es ist natürlich auch möglich, nur einen Detektor zu verwenden, welcher die beiden Strahlungen über ein rasch laufendes Filterrad mit unterschiedlichen Eingangsfiltern separiert.

Die mit 3 bezeichnete Grenzfläche kann für die Anregungsstrahlung oder die von den Sensorschichten emittierte Strahlung transparent sein, sodaß abweichend von der optischen Anordnung nach Fig. 1 auch eine Durchlichtgeometrie verwendet werden kann.

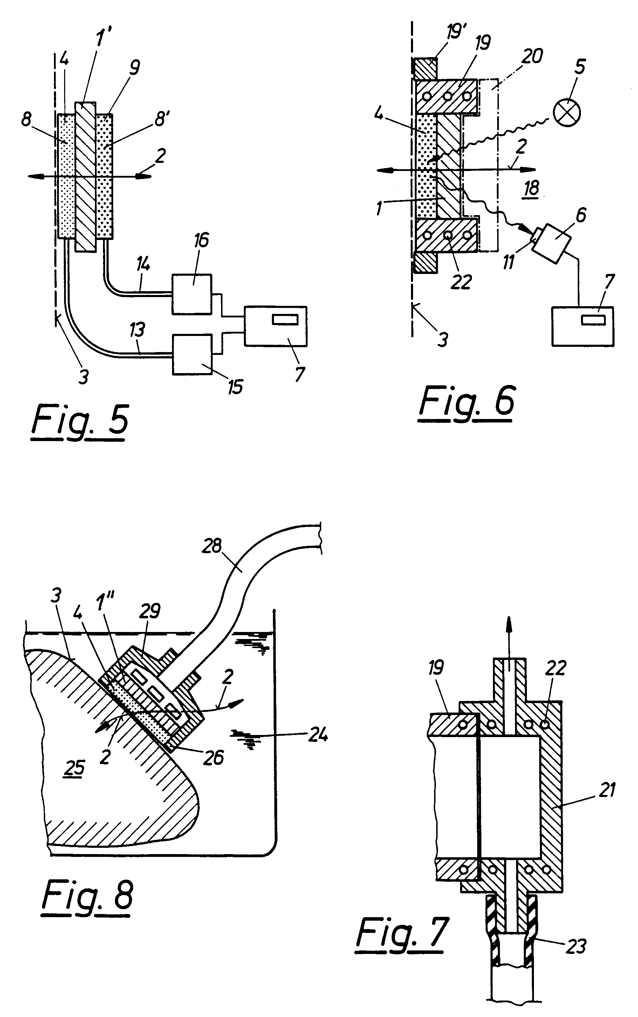

Die Schichtanordnungen nach Fig. 2 oder 4 bilden einen kompletten Sensor zur Bestimmung des Materieflusses ("Fluxsensor"). Bei den Anordnungen nach Fig. 1 oder 3 muß die Materiekonzentration auf einer Seite der Grenzschicht 3 bekannt sein. Je nach den äußeren Gegebenheiten kann bei den unten beschriebenen Ausführungsvarianten nach Fig. 5 bis 10 einer der Fluxsensoren nach Fig. 1 bis 4 verwendet werden.

In der Ausführungsvariante entsprechend Fig. 5 werden die Sensorschichten (Optoden) 4 bzw. 9 bzw. die Indikatoren 8, 8' über seitlich herangeführte Lichtleiter 13 und 14 angeregt. Die Lichtführung innerhalb der Sensorschichten kann dabei beispielsweise durch Totalreflexion erfolgen. Die vorzugsweise zweiarmigen Lichtleiter 13 und 14 sind jeweils mit Anregungs- und Meßeinrichtungen 15 und 16 verbunden, welche ihrerseits an die Auswerteeinrichtung 7 angeschlossen sind. Bei dieser Ausführungsvariante kann die Widerstandsschicht 1' optisch undurchlässig (z.B. als geschwärztes Hydrogel) ausgeführt sein und kann auch für die optische Trennung der beiden Meßstrahlungen verwendet werden, sodaß bei dieser Ausführungsvariante beide Optoden denselben Indikator 8 enthalten können.

Fig. 6 zeigt eine Ausführungsvariante, mit welcher z. B. der $O_2$-Fluß durch die Haut als Grenzfläche 3 gemessen werden kann. Da der $O_2$-Partialdruck in der Umgebungsluft, welche hier als Gasreservoir 18 fungiert, bekannt ist, oder sich aus den Größen Luftdruck, Luftfeuchtigkeit und Temperatur eindeutig bestimmen läßt, ist nur eine $O_2$-Optode bzw. Sensorschicht 4 notwendig, welche in eine an beiden Seiten offene Halterung 19 zusammen mit der Widerstandsschicht 1 seitlich befestigt ist. Die Halterung 19 kann auch - wie mit Bezugszeichen 22 angedeutet - thermostatisiert sein. In einer im Zusammenhang mit Fig. 7 noch ausführlicher beschriebenen Meßanordnung kann auf die Halterung 19 ein gasdichter Verschluß 20 aufgesetzt werden, um den Gasfluß zu unterbinden. Die Halterung 19 weist ein Befestigungselement 19' auf, mit welchem eine Fixierung auf der Haut, beispielsweise durch Kleben oder Anlegen eines Unterdruckes gewährleistet ist.

Aus Fig. 7 ist weiters eine thermostatisierbare Begasungseinrichtung 21 ersichtlich, welche anstelle des Verschlusses 20 ebenfalls auf die Halterung 19 aufsetzbar ist. Die Begasungseinrichtung 21 steht über eine Zuleitung 23 mit einer nicht dargestellten Gasquelle in Verbindung. Mit der in den Fig. 6 und 7 dargestellten Vorrichtung kann beispielsweise der transcutane Sauerstoffpartialdruck der Haut nichtinvasiv gemessen werden.

Eine mögliche Ausführungsvariante ist am Beispiel einer Messung des Ionenflusses in Fig. 8 dargestellt. Z.B. kann damit der Ionenfluß zwischen einer Nährlösung 24 und einem darin aufbewahrten Spenderorgan 25 gemessen werden, wenn auf die Organoberfläche 3 (=Grenzfläche) eine Halterung 26 aufgesetzt wird, welche eine ionenpermeable, optisch durchlässige Widerstandsschicht 1″ und eine zwischen der Widerstandsschicht 1″ und der Organoberfläche angeordnete, ionenpermeable Sensorschicht 4 aufweist. Die Halterung 26 nimmt weiters einen auf die Sensorschicht 4 gerichteten Lichtleiter 28 zum Transport der Anregungs- und Meßstrahlung auf. Zur Aufrechterhaltung des Ionenflusses (z.B. Natrium-, Kalium-, Kalzium- oder Chloridionen etc.) in Richtung der Pfeile 2 ist über die Widerstandsschicht 1″ ein Raum vorgesehen, in welchen die Nährlösung 24 über Öffnungen 29 einströmen kann. Falls das Volumen der Nährlösung groß genug ist, kann deren Ionenkonzentration in guter Näherung als konstant betrachtet werden, sodaß auch hier nur eine Sensorschicht 4 notwendig ist. Es könnte dabei auch eine Sensorschicht, welche gleichzeitig als Widerstandsschicht fungiert, verwendet werden (siehe Fig. 1). Für alle anderen Anwendungsfälle kann beispielsweise eine Anordnung ähnlich Fig. 2 oder 4 mit zwei Sensorschichten 4 und 9 gewählt werden, bei welcher dann auch optisch undurchlässige, ionenpermeable Widerstandsschichten 1″ zulässig sind.

Als Sensorschicht bzw. als Widerstandsschicht kommen im Fall von Elektrolytmessungen vor allem PVC-Schichten mit Ionencarriern in Frage. Damit ist es möglich, folgende Ionen zu vermessen: $Na^+$, $K^+$, $Li^+$, $Mg^{2+}$, $Ca^{2+}$, $Cl^-$ und $NH_4^+$.

Dabei verwendet man z.B. PVC, in dem ein Chromoionophor und zwei Ionencarrier gelöst sind. Ein Ionencarrier transportiert das $H^+$-Ion, der zweite Ionencarrier ist spezifisch für den zu untersuchenden Analyten ($Na^+$, $K^+$,...). Der $H^+$-spezifische Ionencarrier transportiert $H^+$-Ionen zwischen der Probe, bzw. der Widerstandsschicht und dem Chromoionophor, welcher ein pH-Indikator ist und sich in Abhängigkeit der $H^+$-Konzentration in seinen spektralen Eigenschaften verändert. Das $H^+$-Ion wird aus Lösungen mit konstantem pH-Wert und unterschiedlichen Elektrolytkonzentrationen nur dann an den Chromoionophor heran- oder wegtransportiert, wenn sich durch den Transport des Analyten ($Na^+$, $K^+$ ...) die elektrische Ladung der Membran verändert. Voraussetzung für die Funktionstüchtigkeit dieses Sensorprinzips ist der Transport von Analyt und $H^+$-Ion, die Aufrechterhaltung der Elektroneutralität und das Vorliegen eines konstanten PH-Wertes in der Probe.

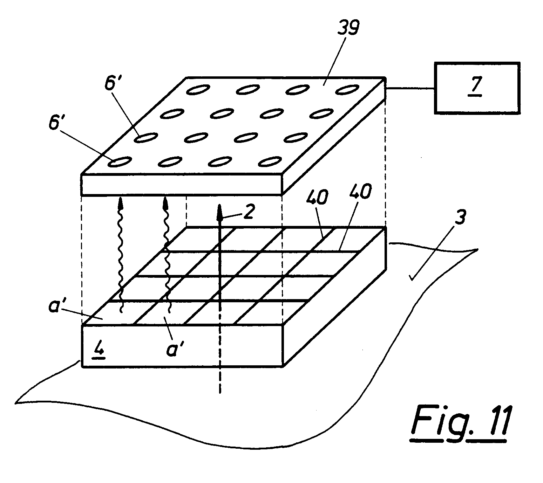

Mit einer in Fig. 9 dargestellten Ausführungsvariante kann die Konzentration eines Agens, beispielsweise eines Enzymsubstrates in einer Probe 32 bestimmt werden. Die Vorrichtung weist einen Reaktionsraum 30 auf, in welchem ein biochemisches Substrat, z.B. ein Enzym vorliegt, welches beispielsweise an der Sensorschicht 4 immobilisiert sein kann. Reagiert nun das Enzymsubstrat einer in den Reaktionsraum 30 eingebrachten Probe 32 mit dem Enzym bzw. der Enzymschicht 31, wobei ein für die enzymatische Reaktion benötigtes Gas (z.B. $O_2$) entlang Pfeil 2 in den Reaktionsraum 30 einströmt, so kann aus dem Gasfluß auf die Konzentration des Enzymsubstrates geschlossen werden. Falls der Gaspartialdruck auf der vom Reaktionsraum 30 abgewandten Seite konstant ist, kann auch hier auf die Sensorschicht 9 verzichtet werden.

Falls in der Probe selbst das für die enzymatische Reaktion benötigte Gas, z.B. Sauerstoff, gelöst ist, muß zur Vermeidung von Meßfehlern entsprechend Fig. 10 zwischen Reaktionsraum 30 und dem als Durchflußzelle ausgeführten Probenraum 33 ein weiterer Fluxsensor, bestehend z.B. aus einer Widerstandsschicht 1′ und Sensorschichten 4′ bzw. 9′, angeordnet sein. Dieser Fluxsensor muß sowohl gasdurchlässig (Pfeil 37) als auch für das zu messende Enzymsubstrat permeabel sein (Pfeil 38). Falls das zu messende Gas im Reaktionsraum 30 homogen verteilt ist und in den Sensorschichten 4 und 4′ der selbe Gaspartikeldruck herrscht, kann beispielsweise auf die Sensorschicht 4′ mit dem Indikator 8″ auch verzichtet werden.

Zur Messung z.B. der Glucosekonzentration kann die Enzymschicht 31 in Fig. 9 oder der Reaktionsraum 30 in Fig. 10 das Enzym Glucoseoxydase (GOD) enthalten, wobei die Sensorschichten 4 und 9 die jeweiligen $pO_2$-Werte zur Bestimmung des $O_2$-Flusses ermitteln.

Die in den Fig. 1 bis 4 dargestellten Vorrichtungen können vom Prinzip her auch zur Messung des Flusses eines Enzymsubstrates beispielsweise durch eine Organoberfläche verwendet werden. Dazu müssen für das zu messende Enzymsubstrat durchlässige Sensor- bzw. Widerstandsschichten verwendet werden. Zur Messung der Enzymkonzentration an den beiden Seiten einer Widerstandsschicht 1 sind z.B. optische Sensorelemente 4 bzw. 9 vorgesehen, welche ein Enzym aus der Gruppe der Oxydasen und der Oxygenasen mit einem dazugehörigen Flavincoenzym (FMN,FAD) aufweisen. Über die Messung der Eigenfluoreszenz des Coenzyms wird in der Auswerteeinheit die jeweilige Konzentration des Enzymsubstrates und aus der Differenz der Konzentrationswerte der Fluß des Enzymsubstrates durch die Grenzfläche 3 bestimmt. Bei Verwendung entsprechender Sensorelemente kann auch eine optische Anordnung ohne Widerstandsschicht, ähnlich jener nach Fig. 2, verwendet werden.

Das vorgestellte Meßprinzip sowie die Meßvorrichtungen eignen sich natürlich auch dafür, bei bekanntem Materiefluß und bekannter Materiekonzentration auf einer Seite einer Grenzfläche die unbekannte Materiekonzentration auf der anderen Seite zu bestimmen.

Weiters kann beispielsweise mit einer Vorrichtung nach Fig. 9 oder 10 auch der $O_2$-Verbrauch einer Zellkultur oder einer mit Bakterien versehenen Nährlösung bestimmt werden.

Um Fehllicht auszuschließen oder unterschiedliche Lichtwege optisch zu trennen, können sowohl die Sensorschichten 4, 9 als auch die Widerstandsschicht 1 pigmentiert oder geschwärzt sein. Beispielsweise können Silikon- oder Hydrogelschichten durch Ruß oder Eisenoxid geschwärzt oder in mikro-disperser Verteilung kolloidale Edelmetallpartikel, z.B. Gold oder Platin, enthalten.

Mit der in Fig. 11 dargestellten Vorrichtung kann die topographische Verteilung des Materieflusses (z.B. $O_2$-Fluß) durch eine Grenzfläche 3 (z.B. Haut) gemessen werden. Dazu dient eine Meßvorrichtung 39, welche die Sensorschicht 4 flächenförmig abtastet. Die Meßvorrichtung 39 kann entweder eine Vielzahl von Detektoren 6' aufweisen, die jeweils einen bestimmten Bereich a' der Sensorschicht 4 zugeordnet sind oder nur einen Detektor, welche die Bereiche a' zeilenartig abtastet. Vorzugsweise kann ein bildgebendes System (z.B. CCD) verwendet werden, welches direkt auf der Sensorschicht 4 aufliegt.

Zur Verringerung der Querdiffusion in der Sensorschicht 4 kann diese in ein Netzwerk 40 mit einem hohen Diffusionskoeffizienten eingebettet sein. Das Netzwerk 40 kann aus Lichtleiter, z.B. für die Einleitung des Anregungslichtet oder aus Metalldraht zur Thermostatisierung der Sensorschicht 4 bestehen.

## Patentansprüche

1. Vorrichtung zur Bestimmung des Materieflusses durch eine Grenzfläche, **dadurch gekennzeichnet**, daß zumindest eine der Grenzfläche (3) zuordenbare Sensorschicht (4; 9) vorgesehen ist, welche dem Materiefluß einen bekannten oder vorbestimmbaren, endlichen Widerstand entgegensetzt, daß in der Sensorschicht (4; 9) ein optischer Indikator (8; 8') zur Bestimmung eines ersten Meßwertes einer von der durchschnittlichen Materiekonzentration in der Sensorschicht (4) abhängigen Größe vorgesehen ist, daß ein zweiter Meßwert dieser Größe an einer Seite der Sensorschicht (4) bekannt oder durch einen weiteren, in einer zweiten Sensorschicht (9) vorliegenden optischen Indikator (8') bestimmbar ist, sowie daß eine Auswerteeinrichtung (7) zur Bestimmung des Materieflusses aus der Differenz der beiden Meßwerte vorgesehen ist.

2. Vorrichtung nach Anspruch 1, **dadurch gekennzeichnet**, daß zumindest eine an der Sensorschicht (4) anliegende bzw. zwischen den beiden Sensorschichten (4; 9) angeordnete Widerstandsschicht (1; 1'; 1″) vorgesehen ist, welche dem Materiefluß einen bekannten oder vorbestimmbaren, endlichen Widerstand entgegensetzt.

3. Vorrichtung nach Anspruch 1 oder 2, **dadurch gekennzeichnet**, daß zur Bestimmung des Gasflusses alle Sensor- (4, 9) bzw. Widerstandsschichten (1; 1') zumindest für das zu messende Gas durchlässig sind, wobei zumindest einer der optischen Indikatoren (8, 8') zur Bestimmung einer vom Gaspartialdruck oder der Gaskonzentration abhängigen Größe geeignet ist.

4. Vorrichtung nach Anspruch 3, **dadurch gekennzeichnet**, daß zur Bestimmung des Flusses zumindest eines der Gase $O_2$, $CO_2$, $H_2$, $NH_3$, Wasserdampf oder Narkosegase durch eine Organoberfläche, z.B. durch die Hautoberfläche, als Grenzfläche (3) eine an beiden Seiten offene, auf die Organoberfläche mit einer Seite aufsetzbare Halterung (19) vorgesehen ist, in welcher die gasdurchlässigen Sensor- (4) bzw. Widerstandschichten (1) angeordnet sind, wobei die Sensorschicht (4) zur Bestimmung des Gaspartialdruckes mit einer Seite an der Organoberfläche (3) anliegt und mit der anderen Seite ggf. unter Zwischenlage einer Widerstandsschicht (1) mit einem Gasreservoir (18) bekannter Gaszusammensetzung verbindbar ist.

5. Vorrichtung nach Anspruch 4, **dadurch gekennzeichnet**, daß die Halterung (19) im Bereich von der der Organoberfläche (3) abgewandten Seite durch einen Verschluß (20) gasdicht verschließbar ist.

6. Vorrichtung nach Anspruch 4, **dadurch gekennzeichnet**, daß als Gasreservoir (18) eine auf die Halterung (19) aufsetzbare, ggf. thermostatisierbare Begasungseinrichtung (21) vorgesehen ist.

7. Vorrichtung nach Anspruch 1 oder 2, **dadurch gekennzeichnet**, daß zur Bestimmung des Ionenflusses alle Sensor- (4) bzw. Widerstandsschichten (1″) zumindest für das zu messende Ion permeabel sind, wobei der zumindest in der einen Sensorschicht (4) vorliegende optische Indikator (8) zur Bestimmung einer von der Ionenkonzentration abhängigen Größe geeignet ist.

8. Vorrichtung nach Anspruch 7, **dadurch gekennzeichnet**, daß die Sensorschicht (4) und ggf . die Widerstandsschicht (1″) einen Ionencarrier für die Messung eines Ions aus der Gruppe $Na^+$, $K^+$, $Li^+$, $Mg^{2+}$, $Ca^{2+}$, $Cl^-$, $NH_4{}^+$ aufweist.

9. Vorrichtung nach Anspruch 3, **dadurch gekennzeichnet**, daß zur Bestimmung des Flusses eines an einer biochemischen Reaktion beteiligten Gases ein Reaktionsraum (30) vorgesehen ist, welcher ein biochemisches Substrat, vorzugsweise ein Enzym, enthält und mit der Sensorschicht (4) sowie zumindest indirekt mit einer Probe (32) in Kontakt steht, welche ein zu bestimmendes Agens, vorzugsweise ein entsprechendes Enzymsubstrat enthält, sowie daß in Abhängigkeit des von der Auswerteeinrichtung (7) festgestellten Wertes für den Gasfluß die davon abhängige Konzentration des Agens in der Probe bestimmbar ist.

10. Vorrichtung nach Anspruch 9 mit einem Probenraum, **dadurch gekennzeichnet**, daß zwischen dem Reaktionsraum (30) und einem Probenraum (33) zumindest eine weitere Sensorschicht (4′, 9′) und ggf. eine weitere Widerstandsschicht (1′) angeordnet sind, wobei die Sensorschicht (4′, 9′) und die Widerstandsschicht (1′) sowohl für das an der biochemischen Reaktion beteiligte Gas als auch für das entsprechende Agens in der Probe (32) durchlässig sind.

11. Vorrichtung nach Anspruch 9 oder 10, **dadurch gekennzeichnet**, daß der Reaktionsraum (30) das Enzym Glucoseoxydase (GOD) zur Messung der Glucosekonzentration einer Probe aufweist und daß jede Sensorschicht (4, 9; 4′, 9′) einen Indikator (8, 8′; 8″) zur Bestimmung des $pO_2$-Wertes aufweist.

12. Vorrichtung nach Anspruch 9 oder 10, **dadurch gekennzeichnet**, daß der Reaktionsraum (30) das Enzym Lactatdehydrogenase oder Lactatoxigenase zur Messung der Lactatkonzentration einer Probe aufweist und daß jede Sensorschicht (4, 9; 4′, 9′) einen Indikator (8, 8′; 8'') zur Bestimmung des $pO_2$-Wertes aufweist.

13. Vorrichtung nach einem der Ansprüche 9 bis 12, **dadurch gekennzeichnet**, daß das biochemische Substrat bzw. das Enzym in einer Schicht (31) vorliegt, weiche an der dem Reaktionsraum (30) zugewandten Seite der Sensorschicht (4) immobilisiert ist.

14. Vorrichtung nach Anspruch 13, **dadurch gekennzeichnet**, daß die Schicht (31) mit dem biochemischen Substrat auf der dem Reaktionsraum (30) zugewandten Seite eine permeabilitätsbestimmende Membran aufweist.

15. Vorrichtung nach Anspruch 1 oder 2, **dadurch gekennzeichnet**, daß zur Bestimmung des Flusses eines Enzymsubstrates alle Sensor- (4; 9) bzw. Widerstandsschichten (1) für die an einer enzymatischen Reaktion beteiligten Enzymsubstrate durchlässig sind, wobei die zumindest eine Sensorschicht (4; 9) zur Messung der Konzentration eines der Enzymsubstrate ein Enzym aus der Gruppe der Oxydasen und Oxigenasen mit dem dazugehörigen Flavincoenzym (FMN, FAD) aufweist, dessen Eigenfluoreszenz von der Konzentration des Enzymsubstrates abhängig ist.

16. Vorrichtung nach einem der Ansprüche 1 bis 14, **dadurch gekennzeichnet**, daß die Sensorschicht (4; 9) einen lumineszenzoptischen Indikator (8; 8′) enthält.

17. Vorrichtung nach einem der Ansprüche 2 bis 14, **dadurch gekennzeichnet**, daß an der Widerstandsschicht (1; 1′; 1″) mindestens ein die Sensorschicht (4; 9) bildender lumineszenzoptischer Indikator (8; 8′) immobilisiert ist.

18. Vorrichtung nach einem der Ansprüche 1 bis 17, **dadurch gekennzeichnet**, daß die Sensorschicht (4; 9) und die Widerstandsschicht (1; 1′; 1″) für die Anregungsstrahlung und/oder die vom Indikator (8; 8′) emittierte Strahlung durchlässig sind.

19. Vorrichtung nach einem der Ansprüche 1 bis 18, **dadurch gekennzeichnet**, daß der optische Indikator (8; 8′) in Nanokapseln angeordnet ist, welche in der Sensorschicht (4; 9) gleichmäßig verteilt sind.

20. Vorrichtung nach einem der Ansprüche 1 bis 19, **dadurch gekennzeichnet**, daß zumindest eine der Sensorschichten (4; 9) zusätzlich einen optischen Indikator zur Messung der Temperatur aufweist.

**21.** Vorrichtung nach einem der Ansprüche 1 bis 20, **dadurch gekennzeichnet**, daß die zumindest eine Sensorschicht (4; 9) sowie eine allfällige Widerstandsschicht (1; 1'; 1") in einer thermostatisierbaren Halterung (19) angeordnet sind.

**22.** Vorrichtung nach Anspruch 21, **dadurch gekennzeichnet**, daß zumindest eine der Sensor- (4; 9) und/oder Widerstandsschichten (1; 1'; 1") aus einem elektrisch leitenden Polymer besteht, welches über elektrische Anschlüsse (35, 36) verfügt, wodurch eine Widerstandsheizung realisierbar ist.

**23.** Vorrichtung nach einem der Ansprüche 1 bis 22, **dadurch gekennzeichnet**, daß eine mit der Auswerteeinrichtung (7) verbundene Meßvorrichtung (39) vorgesehen ist, welche die zumindest eine Sensorschicht (4; 9) zur Erfassung der topographischen Verteilung des Materieflusses flächenförmig abtastet.

**24.** Vorrichtung nach Anspruch 23, **dadurch gekennzeichnet**, daß die Diffusioriseigenschaften der Sensorschicht (4; 9) an jene des zu messenden Objektes angepaßt sind.

**25.** Vorrichtung nach Anspruch 23 oder 24, **dadurch gekennzeichnet**, daß die Sensorschicht (4; 9) in ein die Querdiffusion in der Sensorschicht verhinderndes Netzwerk (40) mit einem hohen Diffusionskoeffizienten eingebettet ist.

**26.** Vorrichtung nach Anspruch 25, **dadurch gekennzeichnet**, daß das Netzwerk (40) aus Lichtleitern besteht.

**27.** Vorrichtung nach Anspruch 25, **dadurch gekennzeichnet**, daß das Netzwerk (40) aus Metalldraht zur Thermostatisierung der Sensorschicht (4; 9) besteht.

**Claims**

**1.** An apparatus for determining the flow of matter passing through a boundary surface, **wherein** is provided at least one sensing layer (4; 9) corresponding to the boundary surface (3) and presenting a known or predetermined, finite resistance to the material flow, and wherein an optical indicator (8; 8') is provided in the sensing layer (4; 9) for obtaining a first measured value of a quantity dependent on the mean concentration of matter in the sensing layer (4), and wherein a second measured value of this quantity is known on one side of the sensing layer (4), or can be determined by means of another optical indicator (8') provided in a second sensing layer (9), and further, wherein an evaluation unit (7) is provided for inferring the material flow from the difference of the two values measured.

**2.** An apparatus as in claim 1, **wherein** at least one resisting layer (1; 1'; 1'') is provided, which is be adjacent to the sensing layer (4), or located between the two sensing layers (4; 9), and which presents a known or predetermined, finite resistance to the material flow.

**3.** An apparatus as in claim 1 or 2, **wherein** for determination of gas flow all sensing (4, 9) and resisting layers (1; 1') are permeable at least to the gas being measured, at least one of the optical indicators (8, 8') being suitable for determining a quantity dependent on partial gas pressure or gas concentration.

**4.** An apparatus as in claim 3, **wherein** for determination of the flow of one or more gases such as $O_2$, $CO_2$, $H_2$, $NH_3$, water vapor, or anaesthetic gases, passing through an interface (3) represented by the surface of an organ, for instance, the skin, a frame (19) is provided, which is open on both sides, one side being placed on the surface of the organ, and which holds the gas-permeable sensing (4) and resisting (1) layers, one side of the sensing layer (4) for determining the partial gas pressure being adjacent to the surface of the organ (3), while the other side is connected to a reservoir (18) containing a gas of known composition, a resisting layer (1) being provided between sensing layer (4) and reservoir (18), if necessary.

**5.** An apparatus as in claim 4, **wherein** the frame (19) is covered with a gas-tight cap (20) on the side of the frame (19) facing away from the surface of the organ (3).

6. An apparatus as in claim 4, **wherein** a gas supply unit (21) is provided as a gas reservoir (18), possibly thermostat-controlled, which may be attached to the frame (19).

7. An apparatus as in claim 1 or 2, **wherein** for determining ionic flow, all sensing (4) and/or resisting (1'') layers are permeable at least to the ion being measured, the optical indicator (8) present in at least one of the sensing layers (4) being suitable for determination of a quantity dependent on ionic concentration.

8. An apparatus as in claim 7, **wherein** the sensing layer (4) and, if present, the resisting layer (1'') has a ionic carrier for measuring an ion from the group of $Na^+$, $K^+$, $Li^+$, $Mg^{2+}$, $Ca^{2+}$, $Cl^-$, $NH_4^+$.

9. An apparatus as in claim 3, **wherein** for determining the flow of a gas participating in a biochemical reaction a reaction chamber (30) is provided, which contains a biochemical substrate, preferably an enzyme, and is in contact with the sensing layer (4) and, at least indirectly, with a sample (32) containing an agent to be determined, preferably a corresponding enzyme reactant, and wherein the concentration of the agent in the sample is determined as a variable depending on the gas flow value obtained from the evaluation unit (7).

10. An apparatus as in claim 9 with a sample chamber , **wherein** at least one further sensing layer (4', 9') and, if necessary, a further resisting layer (1'), is provided between the reaction chamber (30) and a sample chamber (33), the sensing layer (4', 9') and the resisting layer (1') being permeable both to the gas participating in the biochemical reaction and to the corresponding agent in the sample (32).

11. An apparatus as in claim 9 or 10, **wherein** the reaction chamber (30) contains the enzyme glucose oxidase (GOD) for determining the glucose concentration in a sample, and wherein each sensing layer (4, 9; 4', 9') is provided with an indicator (8, 8'; 8'') for determining the $pO_2$ value.

12. An apparatus as in claim 9 or 10, **wherein** the reaction chamber (30) contains the enzyme lactic dehydrogenase or lactic oxygenase for determining the lactate concentration in a sample, and wherein each sensing layer (4, 9; 4', 9') is provided with an indicator (8, 8'; 8'') for determining the $pO_2$ value.

13. An apparatus as in any of claims 9 to 12, **wherein** the biochemical substrate, or rather, the enzyme is provided in a layer (31), which is immobilized on the side of the sensing layer (4) facing the reaction chamber (30).

14. An apparatus as in claim 13, **wherein** the layer (31) containing the biochemical substrate is provided with a membrane of selective permeability on the side facing the reaction chamber (30).

15. An apparatus as in claim 1 or 2, **wherein** for the purpose of determining the flow of an enzyme reactant all sensing (4; 9) and/or resisting (1) layers are permeable to the enzyme reactants participating in an enzymatic reaction, the sensing layer or layers (4; 9) used for measuring the concentration of one of the enzyme reactants containing an enzyme from the group of oxidases and oxygenases and the appropriate flavine coenzyme (FMN, FAD), whose intrinsic fluorescence depends on the concentration of the enzyme reactant.

16. An apparatus as in any of claims 1 to 14, **wherein** the sensing layer (4; 9) contains a luminescence-optical indicator (8; 8').

17. An apparatus as in any of claims 2 to 14, **wherein** at least one luminescence-optical indicator (8; 8') forming the sensing layer (4; 9) is immobilized on the resisting layer (1; 1'; 1'').

18. An apparatus as in any of claims 1 to 17, **wherein** the sensing layer (4; 9) and the resisting layer (1; 1'; 1'') are transparent to the exciting radiation and/or the radiation emitted by the indicator (8; 8').

19. An apparatus as in any of claims 1 to 18, **wherein** the optical indicator (8; 8') is placed in nanocapsules, which are evenly distributed within the sensing layer (4; 9).

**20.** An apparatus as in any of claims 1 to 19, **wherein** at least one of the sensing layers (4; 9) is additionally provided with an optical indicator for temperature measurement.

**21.** An apparatus as in any of claims 1 to 20, **wherein** at least one sensing layer (4; 9) and, if present, a resisting layer (1; 1'; 1'') are arranged in a thermostat-controlled frame (19).

**22.** An apparatus as in claim 21, **wherein** at least one of the sensing (4; 9) and/or resisting (1; 1'; 1'') layers is made of electrically conductive polymer material, which is provided with electrical contacts (35, 36), in order to permit resistance heating.

**23.** An apparatus as in any of claims 1 to 22, **wherein** a measuring device (39) coupled to the evaluation unit (7) is provided, which scans the area of at least one sensing layer (4; 9) to detect the topographical distribution of the material flow.

**24.** An apparatus as in claim 23, **wherein** the diffusion characteristics of the sensing layer (4; 9) are adjusted to those of the object to be measured.

**25.** An apparatus as in claim 23 or 24, **wherein** the sensing layer (4; 9) is embedded in a mesh (40) with high diffusion coefficient, preventing cross-diffusion in the sensing layer.

**26.** An apparatus as in claim 25, **wherein** the mesh (40) consists of optical fibers.

**27.** An apparatus as in claim 25, **wherein** the mesh (40) consists of metal wire used for temperature-control of the sensing layer (4; 9).

**Revendications**

**1.** Dispositif pour la détermination du flux de matière à travers une interface caractérisé, en ce qu'on a prévu au moins une couche de détection (4; 9) associée à l'interface (3), qui oppose une résistance finale connue ou prédéterminable au flux de matière, en ce qu'on a prévu dans la couche détectrice (4; 9) un indicateur optique (8; 8') pour déterminer une premiére valeur de mesure de la concentration moyenne de matière dans la grandeur dépendant de la couche détectrice (4), en ce qu'on connait une deuxième valeur de mesure de cette grandeur, sur une face de la couche détectrice (4) ou on peut la déterminer par un autre indicateur optique (8') présent dans une deuxième couche détectrice (9), et en ce qu'on a prévu un dispositif d'exploitation (7) pur déterminer le flux de matière à partir de la différence des deux valeurs de mesure.

**2.** Dispositif selon la revendication 1 caractérisé en ce qu'on a prévu au moins une couche résistante (1, 1', 1'') située sur la couche détectrice (4) ou placée entre les deux couches détectrices (4, 9), qui oppose une résistance finale connue ou prédéterminable au flux de matière.

**3.** Dispositif selon la revendication 1 ou 2, caractérisé en ce que pour la détermination du flux gazeux toutes les couches détectrices (4, 9) ou résistants (1, 1') sont perméables au moins pour le gaz à mesurer, tandis qu'au moins un des indicateurs optiques (8, 8') est adapté à la détermination d'une grandeur dépendant de la pression partielle du gaz ou de la concentration du gaz.

**4.** Dispositif selon la revendication 3, caractérisé en ce qu'on a prévu pour la détermination du flux d'au moins un des gaz $O_2$, $CO_2$, $H_2$, $NH_3$, vapeur d'eau ou gaz narcotiques à travers une surface d'organe, par exemple à travers la surface de la peau, comme interface (3) une fixation (19) ouverte des deux côtés et pouvant être appuyée par un côté sur la surface d'organe, fixation dans laquelle les couches détectrices (4) perméables aux gaz ou les couches résistantes (1) sont mises en place, la couche détectrice (4) adhérant par une face à la surface d'organe (3) pour la détermination de la pression partielle du gaz et par l'autre face pouvant être reliée éventuellement avec intercalation d'une couche résistante (1) à un réservoir de gaz (18) de composition connue.

**5.** Dispositif selon la revendication 4, caractérisé en ce que la fixation (19) peut être obtenue au niveau de la face éloignée de la surface d'organe (3) par une fermeture (20) étanche aux gaz.

**6.** Dispositif selon la revendication 4, caractérisé en ce que comme réservoir de gaz (18) on a prévu un dispositif d'absorption de gaz (21) applicable sur la fixation (19) et pouvant éventuellement être rendu thermostatique.

**7.** Dispositif selon la revendication 1 ou la revendication 2, caractérisé en ce que pour la détermination du flux ionique toutes les couches détectrices (4) ou résistantes (1'') sont au moins perméables à l'ion à mesurer, l'indicateur optique (8) présent au moins dans une des couches détectrices (4) étant indiqué pour déterminer une grandeur fonction de la caractérisation ionique.

**8.** Dispositif selon la revendication 7, caractérisé en ce que la couche détectrice (4) et éventuellement la couche résistante (1'') ont un agent véhiculaire pour la mesure d'un ion appartenant au groupe $Na^+$, $K^+$, $Li^+$, $Mg^{2+}$, $Ca^{2+}$, $CL^-$, $NH^+_4$.

**9.** Dispositif selon la revendication 3, caractérisé,
- en ce que pour la détermination du flux d'un gaz intéressé dans une réaction biochimique, on a prévu une chambre réactionnelle (30), qui contient un substrat biochimique, de préférence un enzyme et qui est en contact avec la couche détectrice (4) ainsi qu'au moins indirectement avec un échantillon (32) qui contient un agent à déterminer, de préférence un substrat d'enzyme correspondant, et
- en ce qu'en fonction de la valeur fixée par le dispositif d'évaluation (7) pour le flux de gaz la concentration de l'agent chimique en dépendant peut être déterminée dans l'échantillon.

**10.** Dispositif selon la revendication 9, caractérisé en ce qu'entre la chambre réactionnelle (30) et une chambre d'échantillons (33) sont disposés au moins une autre couche détectrice (4', 9') et éventuellement une autre couche résistante (1'), la couche détectrice (4', 9') et la couche résistante (1') étant perméables aussi bien pour le gaz faisant partie de la réaction que pour l'agent correspondant dans l'échantillon (32).

**11.** Dispositif selon la revendication 9 ou la revendication 10, caractérisé
- en ce que la chambre réactionnelle (30) comporte l'enzyme glucose-oxydose (GOD) pour la mesure de la concentration en glucose d'une éprouvette et
- en ce que chaque couche détectrice (4, 9 ; 4', 9') a un indicateur (8, 8', 8'') pour la détermination de la valeur $pO_2$.

**12.** Dispositif selon la revendication 9 ou la revendication 10, caractérisé
- en ce que la chambre réactionnelle (30) comporte l'enzyme lactate-dehydrogénase ou lactateoxygénase pour la détermination de la concentration en lactate d'un échantillon et
- en ce que chaque couche détectrice (4, 9 ; 4', 9') a un indicateur (8, 8', 8'') pour la détermination de la valeur $pO_2$.

**13.** Dispositif selon une des revendications 9 à 12, caractérisé en ce que le substrat biochimique ou l'enzyme est présent dans une couche (31), qui est immobilisée sur la face de la couche détectrice (4) tournée vers la chambre réactionnelle (30).

**14.** Dispositif selon la revendication 13, caractérisé en ce que la couche (31) avec le substrat biochimique est munie d'une membrane déterminant la perméabilité sur la face tournée vers la chambre réactionnelle (30).

**15.** Dispositif selon la revendication 1 ou la revendication 2, caractérisé en ce que pour la détermination du flux d'un substrat d'enzyme toutes les couches détectrices (4, 9) ou résistantes (1) sont perméables pour les substrats d'enzyme intéressés à une réaction enzymatique, l'une des couches détectrices (4, 9) au moins comportant pour la mesure de la concentration d'un des substrats d'enzyme un enzyme du groupe des oxydases et oxygénases avec le Flovincoenzyme (FMN, FAD) qui en fait partie, dont la fluorescence propre est fonction de la concentration du substrat d'enzyme.

**16.** Dispositif selon une des revendications 1 à 14, caractérisé en ce que la couche détectrice (4, 9) contient un indicateur optique de luminescence (8, 8').

**17.** Dispositif selon une des revendication 2 à 14, caractérisé en ce que sur la couche résistante (1, 1', 1'') est immobilisé au moins un indicateur (8, 8') optique de luminescence formant la couche détectrice (4, 9).

**18.** Dispositif selon une des revendication 1 à 17, caractérisé en ce que la couche détectrice (4, 9) et la couche résistante (1, 1', 1'') sont perméables au rayonnement d'excitation et/ou au rayonnement émis par l'indicateur (8, 8').

**19.** Dispositif selon une des revendications 1 à 18, caractérisé en ce que l'indicateur optique (8, 8') est placé dans des nanocapsules, qui sont réparties de façon régulière dans la couche détectrice (4, 9).

**20.** Dispositif selon une des revendication 1 à 19, caractérisé en ce qu'au moins une des couches détectrices (4, 9) a additionnellement un indicateur optique pour la mesure de la température.

**21.** Dispositif selon une des revendications 1 à 20, caractérisé en ce qu'au moins l'une des couches détectrices (4, 9) ainsi qu'une couche résistante (1, 1', 1'') éventuelle sont placées dans une fixation (19) pouvant être thermostatisée.

**22.** Dispositif selon la revendication 21, caractérisé en ce qu'au moins une des couches détectrices (4, 9) et/ou une des couches résistantes (1, 1', 1'') est composée d'un polymère conducteur électrique, qui dispose de raccordements électriques (35, 36) et grâce auquel un chauffage par résistance peut être réalisé.

**23.** Dispositif selon une des revendications 1 à 22, caractérisé en ce qu'un des dispositifs de mesure (39) relié au dispositif d'évaluation (7) est prévu, qui explore de façon planiforme au moins une couche détectrice (4, 9) pour déterminer la répartition topographique du flux de matière.

**24.** Dispositif selon la revendication 23, caractérisé en ce que les propriétés de diffusion de la couche détectrice (4, 9) sont adaptées à celles de l'objet à mesurer.

**25.** Dispositif selon la revendication 23 ou la revendication 24, caractérisé en ce que la couche détectrice (4, 9) est insérée avec un coefficient de diffusion élevé dans un réseau (40) empêchant la diffusion transversale dans la couche détectrice.

**26.** Dispositif selon la revendication 25 caractérisé en ce que le réseau (40) est composé de câbles fibre optique.

**27.** Dispositif selon la revendication 25, caractérisé en ce que le réseau (40) est composé de fil métallique pour la thermostatisation de la couche détectrice (4, 9).

Fig. 1

Fig. 3

Fig. 4

Fig. 2

Fig. 9

Fig. 10

15

_Fig. 5_

_Fig. 6_

_Fig. 8_

_Fig. 7_

Fig. 11